# EUROPÄISCHE PATENTANMELDUNG

(11) **EP 3 578 970 A1**
(43) Veröffentlichungstag der Anmeldung: **11.12.2019**
(21) Anmeldenummer: 19177973.5
(22) Anmeldetag: 03.06.2019
(51) Int. Cl.: G01N 33/15, G01B 21/00, G01N 3/08

(54) **VERFAHREN ZUM AUTOMATISCHEN ERMITTELN VON GRENZWERTEN VON MINDESTENS EINEM PARAMETER VON KLEINVOLUMIGEN SCHÜTTGÜTERN EINES BESTIMMTEN TYPS**

(30) Priorität: 04.06.2018 DE 102018113220
(71) Anmelder: Kraemer, Thilo, 64291 Darmstadt (DE)
(72) Erfinder: Kraemer, Thilo, 64291 Darmstadt (DE)
(74) Vertreter: Hamel, Armin

(57) **Zusammenfassung**

Die Erfindung betrifft Verfahren zum automatischen Ermitteln von Grenzwerten von mindestens einem Parameter von kleinvolumigen Schüttgütern eines ersten Typs, folgende Schritte aufweisend:
- in einem ersten Schritt wird ein erstes Schüttgut eines ersten Typs in einem Prüfgerät bereitgestellt;
- in einem zweiten Schritt wird ein erster Parameter des ersten Schüttguts vermessen; und
- in einem dritten Schritt wird der erste Parameter als erster Grenzwert in dem Prüfgerät hinterlegt.

## Beschreibung

Die vorliegende Erfindung betrifft ein Verfahren zum automatischen Ermitteln von Grenzwerten von mindestens einem Parameter von kleinvolumigen Schüttgütern eines bestimmten Typs sowie ein Prüfgerät zum Durchführen des Verfahrens.

Mit einem Prüfgerät können bestimmte Parameter von kleinvolumigen Schüttgütern, zum Beispiel Tabletten oder Körner, gemessen werden. Zu diesen Parametern gehören die Breite, die Länge, die Masse, die Härte sowie die Qualität der Schüttgüter.

Ein solches Prüfgerät ist beispielsweise aus DE 20 2015 101 878 U1 bekannt. Damit jedoch ein Schüttgut eines bestimmten Typs, zum Beispiel ein Oblong, vermessen werden kann, müssen in dem Prüfgerät bestimmte Grenzwerte zu jedem Parameter des Schüttguts hinterlegt werden.

Bisher mussten die Grenzwerte für bestimmte Parameter von einer Person einzeln in das Prüfgerät eingepflegt werden. Dies war insbesondere deshalb zeitaufwändig, weil die Parameter zuvor ermittelt werden mussten.

Aufgabe der vorliegenden Erfindung ist es deshalb, ein Verfahren bereitzustellen, mit dem diese Grenzwerte schnell und damit auch kostengünstig ermittelt und bereitgestellt werden können.

Diese Aufgabe wird nach den Merkmalen des Patentanspruchs 1 gelöst.

Somit wird ein Verfahren zum automatischen Ermitteln von Grenzwerten von mindestens einem Parameter von kleinvolumigen Schüttgütern eines bestimmten Typs bereitgestellt (= Autokonfiguration), das folgende aufeinanderfolgende Schritte aufweist.

Zuerst wird ein erstes Schüttgut eines ersten Typs in einem Prüfgerät bereitgestellt (Schritt (i)). In einem zweiten Schritt (Schritt (ii)) wird ein erster Parameter des ersten Schüttguts vermessen. In einem dritten Schritt (Schritt (iii)) wird der ermittelte erste Parameter als erster Grenzwert in dem Prüfgerät hinterlegt. Von Vorteil ist dabei, dass das Prüfgerät diesen ersten Parameter, zum Beispiel die Masse des Schüttguts, automatisch misst und diesen als Grenzwert hinterlegt, was zu einer enormen Zeitersparnis führt.

In einer bevorzugten Ausführungsform wird, nachdem der erste Parameter als erster Grenzwert hinterlegt wurde, mindestens ein weiterer Parameter des ersten Schüttguts vermessen und anschließend dieser mindestens eine weitere Parameter als weiterer Grenzwert hinterlegt. Es können somit für dieses erste Schüttgut nacheinander verschiedene Parameter vermessen werden und diese als weitere Grenzwerte hinterlegt werden. Es ist somit möglich, alle erforderlichen Parameter als Grenzwerte für ein Schüttgut des ersten Typs zu erhalten und diese in dem Prüfgerät zu hinterlegen.

In einer weiteren bevorzugten Ausführungsform wird mindestens ein weiteres Schüttgut des ersten Typs bereitgestellt, und es werden die Schritte (ii) und (iii) durchgeführt. Von diesem mindestens einen weiteren Schüttgut wird somit ebenfalls ein Grenzwert des ersten Parameters erhalten. Vorteilhaft ist dabei, dass aus diesen Grenzwerten ein Mittelwert (= gemittelte Grenzwerte) gebildet werden kann.

Diese Mittelwerte des ersten Parameters werden ebenfalls in dem Prüfgerät hinterlegt. Vorteilhaft ist, wenn 10 bis 20 Schüttgüter des ersten Typs vermessen werden und daraus ein Mittelwert gebildet wird. Durch den so gebildeten Mittelwert fallen mögliche Ausreißer weniger ins Gewicht.

Bevorzugt wird von dem mindestens einen weiteren Schüttgut mindestens ein weiterer Parameter vermessen und anschließend dieser mindestens eine weitere Parameter als weiterer Grenzwert hinterlegt. Wurde zum Beispiel als erster Parameter die Masse vermessen, können als weitere Parameter die Dicke, die Breite, die Härte und/oder die Qualität (worunter auch die chemische Zusammensetzung fällt) der Schüttgüter nacheinander vermessen werden. Es werden somit weitere Parameter von weiteren Schüttgütern vermessen, um daraus für jeden Parameter einen Mittelwert zu bilden. Von Vorteil ist, dass die Messung der Parameter und die Bildung der Mittelwerte der hinterlegten Grenzwerte mittels einer Software vollautomatisch und daher auch sehr schnell erfolgt. Vorzugsweise werden für jeden Parameter 10 bis 20 Schüttgüter nacheinander vermessen.

Schließlich betrifft die Erfindung auch das Prüfgerät zum Durchführen des Verfahrens.

Dabei wird auch explizit vorgeschlagen, mehrere Merkmale der einzelnen beschriebenen Ausführungsformen untereinander zu kombinieren.

Das Verfahren dient folglich zum automatischen Ermitteln von Grenzwerten von mindestens einem Parameter von kleinvolumigen Schüttgütern eines bestimmten Typs, das heißt eines Schüttguts mit ganz bestimmten Parametern. In einem ersten Schritt wird ein erstes Schüttgut eines ersten Typs in dem Prüfgerät bereitgestellt (Schritt (i)). In einem zweiten Schritt (Schritt (ii)) wird ein erster Parameter des ersten Schüttguts vermessen. In einem dritten Schritt (Schritt (iii)) wird der erste Parameter durch eine in dem Prüfgerät bereitgestellte Software als erster Grenzwert in dem Prüfgerät hinterlegt.

Nachdem der erste Parameter als erster Grenzwert hinterlegt wurde, wird mindestens ein weiterer Parameter des ersten Schüttguts vermessen und anschließend dieser mindestens eine weitere Parameter durch die Software als weiterer Grenzwert hinterlegt. Es können somit für dieses erste Schüttgut nacheinander mehrere unterschiedliche Parameter vermessen werden und diese als Grenzwert hinterlegt werden. Nachdem also zum Beispiel die Masse des ersten Schüttguts ermittelt wurde, kann im Anschluss daran die Härte, die Breite, die Dicke und/oder die Qualität des ersten Schüttguts ermittelt werden.

Alle Parameter des Schüttguts werden somit durch Messungen in einer Messstation erhalten. Dabei kann die Masse durch wiegen des Schüttguts erhalten werden. Die Härte, die Dicke und die Breite können in einer Bruchkammer des Prüfgeräts gemessen werden, wie dies beispielsweise in DE 20 2015 101 878 U1 beschrieben ist. Die Qualität des Schüttguts wird vorzugsweise durch eine NIR-Messung erhalten. Es ist somit möglich, alle erforderlichen Parameter als Grenzwerte zu erhalten und diese in dem Prüfgerät zu hinterlegen.

Vorteilhaft ist, wenn mindestens ein weiteres Schüttgut des ersten Typs bereitgestellt wird und anschließend die Schritte (ii) und (iii) durchgeführt werden. Von diesem mindestens einen weiteren Schüttgut wird somit ebenfalls ein Grenzwert des ersten Parameters erhalten. Vorteilhaft ist dabei, dass aus diesen Grenzwerten des ersten Parameters ein Mittelwert gebildet werden kann. Die Bildung des Mittelwerts erfolgt ebenfalls über die in dem Prüfgerät vorgesehene Software.

Von diesen hinterlegten Grenzwerten des ersten Parameters werden Mittelwerte gebildet und diese als gemittelte Grenzwerte in dem Prüfgerät hinterlegt. Vorteilhaft ist, wenn 10 bis 20 Schüttgüter des ersten Typs vermessen werden und daraus ein Mittelwert gebildet wird. Durch diesen Mittelwert fallen mögliche Ausreißer weniger ins Gewicht.

Bevorzugt wird von dem mindestens einen weiteren Schüttgut mindestens ein weiterer Parameter vermessen und anschließend dieser mindestens eine weitere Parameter als weiterer Grenzwert hinterlegt. Wurde zum Beispiel als erster Parameter die Masse vermessen, können im Anschluss daran die Dicke, die Breite, die Härte und/oder die Qualität (zum Beispiel die chemische Zusammensetzung) der Schüttgüter ermittelt werden. Es werden somit weitere Parameter von weiteren Schüttgütern vermessen, um daraus mittels der Software schließlich für jeden Parametertyp einen Mittelwert zu erhalten. Vorzugsweise werden für jeden Parameter 10 bis 20 Schüttgüter nacheinander vermessen.

Da die Messungen der einzelnen Parameter für jedes Schüttgut des ersten Typs automatisch erfolgen, ermittelt die Software des Prüfgeräts somit die entsprechenden Grenzwerte und damit auch die daraus berechneten Mittelwerte für diesen bestimmten Parameter selbst, womit das Prüfgerät eine Autokonfiguration durchführt. Das Prüfgerät wurde also durch diese Autorkonfiguration hinsichtlich eines Schüttguts eines ersten Tys kalibriert.

Nachdem die Autorkonfiguration für das Schüttgut des ersten Typs durchgeführt worden ist, kann natürlich auch eine Autorkonfiguration für ein Schüttgut eines anderen Typs durchgeführt werden.

Durch diese Autorkonfiguration werden so Basiswerte für ein Schüttgut eines bestimmten Typs erhalten, die für weitere Messungen verwendet werden können. Das Prüfgerät wird also durch diese Autorkonfiguration hinsichtlich eines Schüttguts eines bestimmten Typs kalibriert. Werden Messungen an Schüttgütern dieses bestimmten Typs durchgeführt, so kann das Prüfgerät anhand dieser Basiswerte erkennen, ob das entsprechende Schüttgut den Erfordernissen genügt.

Es versteht sich, dass die Grenzwerte für einen bestimmten Parameter von einem Benutzer bei Bedarf noch zusätzlich optimiert werden können.

Vorteilhaft ist dabei auch, dass die ermittelten Grenzwerte bzw. die ermittelten Mittelwerte für die Grenzwerte auch für die Kalibrierung anderer Geräte verwendet werden können.

## Patentansprüche

1. Verfahren zum automatischen Ermitteln von Grenzwerten von mindestens einem Parameter von kleinvolumigen Schüttgütern eines bestimmten Typs, folgende Schritte aufweisend:
(i) in einem ersten Schritt wird ein erstes Schüttgut eines ersten Typs in einem Prüfgerät bereitgestellt;
(ii) in einem zweiten Schritt wird ein erster Parameter des ersten Schüttguts vermessen; und
(iii) in einem dritten Schritt wird der erste Parameter als erster Grenzwert in dem Prüfgerät hinterlegt.

2. Verfahren nach Anspruch 1, **dadurch gekennzeichnet, dass** nach Schritt (iii) mindestens ein weiterer Parameter des ersten Schüttguts vermessen wird und anschließend dieser mindestens eine weitere Parameter als weiterer Grenzwert hinterlegt wird.

3. Verfahren nach Anspruch 1, **dadurch gekennzeichnet, dass** mindestens ein weiteres Schüttgut des ersten Typs bereitgestellt wird und für jedes weitere Schüttgut die Schritte (ii) und (iii) durchgeführt werden.

4. Verfahren nach Anspruch 3, **dadurch gekennzeichnet, dass** von dem mindestens einen weiteren Schüttgut mindestens ein weiterer Parameter vermessen wird und anschließend dieser mindestens eine weitere Parameter als weiterer Grenzwert hinterlegt wird.

5. Verfahren nach Anspruch 1 bis 4, **dadurch gekennzeichnet, dass** von den hinterlegten Grenzwerten hinsichtlich eines bestimmten Parameters Mittelwerte gebildet werden und diese Mittelwerte in dem Prüfgerät hinterlegt werden.

6. Prüfgerät zum Durchführen des Verfahrens nach den Patentansprüchen 1 bis 5.
